# EUROPEAN PATENT SPECIFICATION

(11) **EP 4 618 889 B1**
(45) Date of publication and mention of the grant of the patent: **28.10.2026**
(21) Application number: 23828892.2
(22) Date of filing: 20.11.2023
(51) Int. Cl.: A61C 1/16, A61B 1/00, A61C 9/00

(54) **INTRAORAL SCANNER SLEEVE**
INTRAORALE SCANNERHÜLSE
MANCHON DE SCANNER INTRA-BUCCAL

(30) Priority: 21.11.2022 US 202263427095 P
(43) Date of publication of application: 24.09.2025
(73) Proprietor: Align Technology, Inc., San Jose, CA 95134 (US)
(72) Inventor: SHALEV, Ariel, San Jose, California 95134 (US); LEVY, Raphael, San Jose, California 95134 (US); GORFINKEL, Roee, San Jose, California 95134 (US); VERKER, Tal, San Jose, California 95134 (US); GORDON, Benny, San Jose, California 95134 (US); MAKMEL, Nir, San Jose, California 95134 (US); ISHAY, Eran, San Jose, California 95134 (US)
(74) Representative: Mathys & Squire
(86) International application number: PCT/US2023/080484
(87) International publication number: WO 2024/112639

(56) References cited:
- WO-A1-2015/128717
- WO-A1-2021/021854
- US-S- D 774 193

## Description

### CLAIM OF PRIORITY

This patent application claims priority to U.S. provisional patent application no. 63/427,095, titled "INTRAORAL SCANNER SLEEVE," filed on November 21, 2022.

### BACKGROUND

Many dental and orthodontic procedures can benefit from accurate imaging (including, but not limited to accurate three-dimensional, 3D, imaging, 2D imaging, surface scanning, florescent scanning, etc.) to provide digital descriptions of a patient's dentation and intraoral cavity. An intraoral scanner may provide such imaging. Typically an intraoral scanner may include a hand-held sensing component for scanning within the patient's oral cavity. The hand-held component may be referred to as a wand, and may include one or more windows for transmitting and/or receiving light to form images from within the patient's oral cavity.

Because the intraoral scanners may be inserted at least partially into the patient's mouth, a protective element, referred to herein as a sleeve or as a protective sleeve, may be used with the wand. The sleeve can act as barrier between the wand and the patient to protect the patient from cross-contamination. Thus, the sleeve may be removable from the wand so that the sleeve can be replaced before using the wand with the next patient. However, it has proven difficult to manufacture sleeves that provide sufficient seal against contamination and securely couple with the wand of the intraoral scanner within acceptably high tolerances. In addition, the optical qualities and the shape and size of the sleeves may affect the performance of the intraoral scanner. For example, if the sleeve does not fit on the intraoral scanner properly or does not have good optical transmission properties, the intraoral scanner will not obtain a good scan of the patient's mouth, resulting in inaccurate scan results.

WO2015128717A1 shows a sleeve shaped secondary housing for protecting an intraoral scanner, a transparent element inside the housing lays over an aperture and support extending from the housing on its outer surface hold the transparent element in place.

Described herein are methods and apparatuses, including protective sleeves, systems including protective sleeves, and methods of using them to address these problems and that may enhance the safety and functionality of intraoral scanners.

### SUMMARY OF THE DISCLOSURE

The subject-matter of the present invention is a sleeve for an intraoral scanner as defined in claim 1.

The apparatuses and methods described herein may relate to protective sleeves for optical scanners, and particularly for intraoral scanner that may be useful in scanning the intraoral cavity. A sleeve for an intraoral scanner typically acts as a barrier to prevent biological contamination of the intraoral scanner, e.g., and in particular the wand of the intraoral scanner, including preventing cross contamination between patients. These sleeves described herein may preserve the optical performance of the scanner while also providing a physical protection to the scanner itself. The sleeve may be referred to as a barrier, a jacket, a pouch, a cover, etc. The sleeves described herein may cover the entire intraoral scanner (e.g., the wand and/or connecting cable) or only the tip of the scanner (e.g., the wand portion) to prevent contamination.

The sleeves described herein may be configured to include one or more internal ramps to position and/or secure the scanner of the intraoral scanner in position. The sleeves described herein may optionally not include a ramp, may include an integrated ramp, or may include a separate, attachable (and in some cases detachable) ramp. These ramps may be modular. In some examples, sleeves with modular (e.g., separately attachable) ramps allow for simpler mold construction and higher reliability of the sleeve, particularly when the sleeve is injection molded.

In some examples the sleeves described herein may include a window portion having a transparent cover that is configured and adapted to be held, and in some cases secured, a precise distance from the imaging window or opening of the scanner (e.g., wand) of the intraoral scanner. This distance may be, for example, between 0.05 and 0.3 mm (e.g., between 0.1 and 0.25 mm, between 0.18 and 0.22 mm, etc.). The transparent cover may have a uniform thickness along its length. The variance of this thickness across the length of the transparent cover (at least over the region through which imaging will occur) may be 0.8% or less (e.g., 0.7% or less, 0.6% or less, 0.5% or less, 0.4% or less, 0.3% or less, 0.2% or less, 0.1% or less, 0.08% or less, 0.05% or less, etc.). The transparent cover may have a transparency that is 86% transparent or higher (e.g., 87% or more, 88% or more, 89% or more, 90% or more 91% or more, 92% or more, etc.) to the visible light wavelengths used by the intraoral scanner. In general, the transparent covers may provide a very low distortion of the transmitted wave front from the intraoral scanner.

In some examples, the sleeve may be advantageously configured so that the transparent cover is held within a seating region (e.g., a window having a lip) on an outer surface of the sleeve, which may be recessed into the outer surface. This configuration may allow the sleeve to be secured over the imaging opening (or window) of the scanner of the intraoral scanner at a precisely controlled and uniform spacing, while preventing deformation of the transparent cover during operation. This configuration may also prevent scratching or harm to the transparent cover when inserting and/or operating the scanner. The transparent cover may be secured to the hollow body of the sleeve in any appropriate manner. For example, the transparent cover may be ultrasonically welded to the hollow body. In some examples, it may be particularly helpful to ultrasonically weld the transparent cover to form a seal which is stable over time and is not sensitive to transportation conditions and high temperatures.

In some examples the transparent cover may be integrally formed with the hollow body of the sleeve. For example the hollow body and transparent cover may be formed of a clear material as a single piece that does not require assembly of the transparent cover into the body of the sleeve.

In general, any of these apparatuses may include a more rigid distal body that secured over the scanner (e.g., wand) and includes the transparent widow a less rigid (e.g., highly flexible) extension portion that may extend proximally from the more rigid distal sleeve portion. The extension portion may be referred to as a sleeve extension, and may be formed of the same material as the distal, more rigid, sleeve body or it may be formed of a different material. The sleeve extension may be attached to the sleeve body by an adhesive, a weld, a tape, etc. In some examples the sleeve extension is attached under the more distal sleeve body. The sleeve extension may be applied over the scanner (e.g., wand and/or cord) and the sleeve body may be applied over the sleeve extension. In some cases the sleeve extension is not sealed to the distal sleeve body, but is worn over it. In some examples the sleeve extension is sealed over and/or around the sleeve body. For example, the sleeve may include a sleeve extension (e.g., sheath) that is welded (e.g., laser welded) directly onto the sleeve body. In some cases the sleeve extension may be applied over the sleeve body or may be attached to an outside region of the sleeve body. In some examples the sleeve body and sleeve extension are made of a single material and does not require a double injection processes.

For example, described herein are apparatuses, including sleeves, intraoral scanning systems to use these sleeves, and methods of using the sleeves. Generally described herein are sleeves for an intraoral scanner that are configured to securely couple with an intraoral scanner (e.g., wand, or wand and cord). These sleeves may guide and direct the distal end of a wand of an intraoral scanner into the sleeve securely without risking damage to the wand of the intraoral scanner and/or the transparent cover over the window of the sleeve.

For example, described herein are sleeves (e.g., protective sleeves) for use with an intraoral scanner that include: a hollow body having a distal end and a proximal end, wherein the distal end is tapered and closed and the proximal end is open to receive an intraoral scanner; a window opening on a lateral side of the distal end, wherein the window opening comprises a recessed outer lip formed into the hollow body so that the recessed outer lip is recessed into an outer surface of the hollow body; and a transparent cover over the window opening wherein the transparent cover is seated in the recessed outer lip.

As mentioned above, any of these sleeves may include a ramp within the hollow body. The ramp may be positioned within the hollow body proximal to the window opening, and configured to guide a distal end of the intraoral scanner away from the window opening as the intraoral scanner is inserted into the sleeve. In some examples the ramp is adhesively secured to a wall of the hollow body. The ramp may be repositionable.

For example, described herein are sleeves for an intraoral scanner that may include: a window opening on a lateral side of the distal end, wherein the window opening comprises a recessed outer lip formed into the hollow body so that the recessed outer lip is recessed into an outer surface of the hollow body; a transparent cover over the window opening wherein the transparent cover is seated in the recessed outer lip; and a ramp within the hollow body, wherein the ramp is positioned within the hollow body proximal to the window opening, and configured to guide a distal end of the intraoral scanner away from the window opening as the intraoral scanner is inserted into the sleeve.

In some examples a sleeve for an intraoral scanner includes: a window opening on a lateral side of the distal end, wherein the window opening comprises a recessed outer lip formed into the hollow body so that the recessed outer lip is recessed into an outer surface of the hollow body; a transparent cover over the window opening wherein the transparent cover is seated in the recessed outer lip; and a foam gasket at least partially circumferentially around the window opening between the transparent cover and the recessed outer lip, wherein the transparent cover is secured against the foam gasket.

Any of the sleeves described herein may include a gasket at least partially circumferentially around the window opening between the transparent cover and the recessed outer lip, wherein the transparent cover is secured against the gasket. The gasket may comprise a foam material.

In general, these apparatuses may be dimensioned for compact and lightweight operation. For example, the sleeves described herein may be configured to have a tapered distal end has a height of between 10 and 16 mm.

Any of these apparatuses may include one or more an antireflective (AR) coatings. In particular, these apparatuses may include an antireflective coating on one or both an inner and outer surface of the transparent cover. Also described herein are methods of forming the AR coating, for example, by dip coating, spraying, etc.

In any of these apparatuses, the sleeve may include one or more projections from a periphery of the window into the window opening configured to impinge into a field of view of the intraoral scanner when the intraoral scanner is engaged with the sleeve.

As mentioned, any of these sleeves may include a sleeve extension that may be more flexible than the sleeve body. In some examples the apparatus incudes a sleeve extension extending from the proximal end of the elongate and hollow body. In some examples the sleeve extension is configured to invert over itself when pulled distally. In some examples the sleeve extension is applied and is configured to be inserted into the sleeve body. In some examples the sleeve extension is configured to extend over all or a portion of the sleeve body.

In general, the sleeve body and the sleeve extension may be formed of any appropriate material. In some examples the sleeve body and the sleeve extension are formed of the same material. For example, the sleeve may be formed of a polyethylene material. The sleeve body and the sleeve extension may be attached to each other; in some examples the sleeve body is sealed to the sleeve extension.

The sleeve may include an adhesive seal around the window opening, sealing the transparent cover to the window opening. As mentioned above, the transparent cover may be ultrasonically welded to the hollow body.

In some examples the transparent cover is flush with an outer surface of the hollow body. Alternatively, in some examples the transparent cover extends slightly proud of the outer surface of the hollow body.

Any of the transparent windows may include one or more anti-fogging or de-fogging components, including (but not limited to) coatings or layers for anti-fogging/defogging).

The sleeves described herein may be configured to provide a seal and protection against contamination of the wand so that the same wand may be used with different patients by exchanging sterile sleeves between patients. Any of these sleeves may be configured to seal to greater than 90 kPa.

In general, the wand may be inserted into the sleeve and in some examples may be guided within the inside of the sleeve (e.g., using an internal guiding structure, e.g., ramp, or other guiding feature within the sleeve) so that the imaging sensor(s) of the wand are aligned and positioned correctly and predictably relative to the window opening and transparent cover of the sleeve. In general, the imaging window of the wand (which may be open or may include a cover, e.g., transparent wand cover) may be positioned either against the transparent cover of the sleeve or within a known tolerance of, e.g., about 0.2 mm (e.g., between 0.5 mm and 0.01 mm, between about 0.3 mm and 0.05 mm, between 0.35 mm and 0.1 mm, etc.). In particular, the internal guiding structure features described herein may be configured to position the wand in a fixed relation to the window opening and transparent cover.

As mentioned, described herein are sleeves including a sealing frame that may secure the transparent cover of the sleeve over the window opening. For example, a sleeve for an intraoral scanner may include: an elongate and hollow body having a distal end and a proximal end, wherein the distal end is tapered and closed and the proximal end is open to receive an intraoral scanner; a window opening on a lateral side of the distal end; a transparent cover over the window opening; a sealing frame coupled to an outside of the elongate and hollow body so that the transparent cover is sandwiched between a periphery of the window opening and a sealing frame window opening; and an adhesive channel peripherally at least partially around the sealing frame window opening holding an adhesive securing the transparent cover within the window opening.

In some examples, the sleeves may be formed with a living hinge, as mentioned above. For example, a sleeve for an intraoral scanner may include: an elongate and hollow body having a distal end and a proximal end, wherein the distal end is tapered and closed and the proximal end is open to receive an intraoral scanner; a window opening on a lateral side of the distal end; a transparent cover over the window opening; wherein the elongate and hollow body comprises a clamshell body having a living hinge at one side (e.g., a longitudinal side or the distal end) of the elongate and hollow body, further wherein a lateral seam on one or both sides of the elongate and hollow body is sealed closed.

### BRIEF DESCRIPTION OF THE DRAWINGS

The novel features of the invention are set forth in the claims that follow. A better understanding of the features and advantages of the present invention will be obtained by reference to the following detailed description and the accompanying drawings of which:
FIG. 1 is an example of one variations of an intraoral scanner as described herein.
FIG. 2A illustrates the use of a protective sleeve with the example of the intraoral scanner as described herein.
FIG. 2B illustrates the use of another example of a protective sleeve with an intraoral scanner as described herein.
FIG. 3A illustrates one example of a protective sleeve for an intraoral scanner.
FIG. 3B shows another example of a protective sleeve for an intraoral scanner as described herein.
FIGS. 4A and 4B show examples of top perspective section and bottom perspective section views of one example of a protective sleeve including an integral internal guiding structure (e.g., ramp) as described herein.
FIGS. 4C-4E schematically illustrate cross-sections through examples of internal guiding structures as described herein.
FIGS. 4F and 4G schematically illustrate cross-sections through examples of internal guiding structures as described herein.
FIGS. 5A and 5B illustrate insertion of a wand of an intraoral scanner into a sleeve including in integrated internal guiding structure as described herein.
FIG. 6 is an example of a sleeve include a sleeve extension for use with a wand of an intraoral scanner as described herein.
FIG. 7 is an example of the sleeve of FIG. 6 with a wand of an intraoral scanner inserted into the sleeve.
FIGS. 8A-8B show a comparison between examples of sleeves with internal (FIG. 8A) or external (FIG. 8B) sleeve extensions.
FIGS. 9A-9B illustrate an example of a sleeve including a sleeve extension that is inserted internal to the sleeve. FIG. 9B shows an exploded view of FIG. 9A.
FIG. 10A-10B illustrates an example of a sleeve including a sleeve extension that is external to the sleeve. FIG. 10B shows an exploded view of FIG. 10A.
FIG. 11A illustrates the attachment of a sealing frame over a transparent cover of a sleeve in one example of a sleeve.
FIG. 11B shows the assembled sleeve of FIG. 11A with the sealing frame secured over the transparent cover.
FIG. 12 is an example of a sleeve including snaps within the perimeter of the window region for securing a transparent cover.
FIG. 13 shows a section through a sleeve similar to that shown in FIG. 12 in which a wand of an intraoral scanner has been inserted into the sleeve.
FIG. 14 shows a distal of another example of a sleeve similar to the one shown in FIG. 12 with the transparent cover inserted and a seal applied (e.g., an adhesive seal) around the perimeter of the window opening.
FIGS. 15A-15D illustrate the assembly of another example of a sleeve, in which the transparent cover is applied from the outside of the sleeve and secured in place using a heat press.
FIG. 16 shows another example of a sleeve configured to have an externally applied window.
FIG. 17 is an exploded view of a sleeve having an externally applied window.
FIGS. 18A-18B show another example of a sleeve having an externally applied window.
FIGS. 19A and 19B show an example of a sleeve configured as a clamshell having a living hinge.

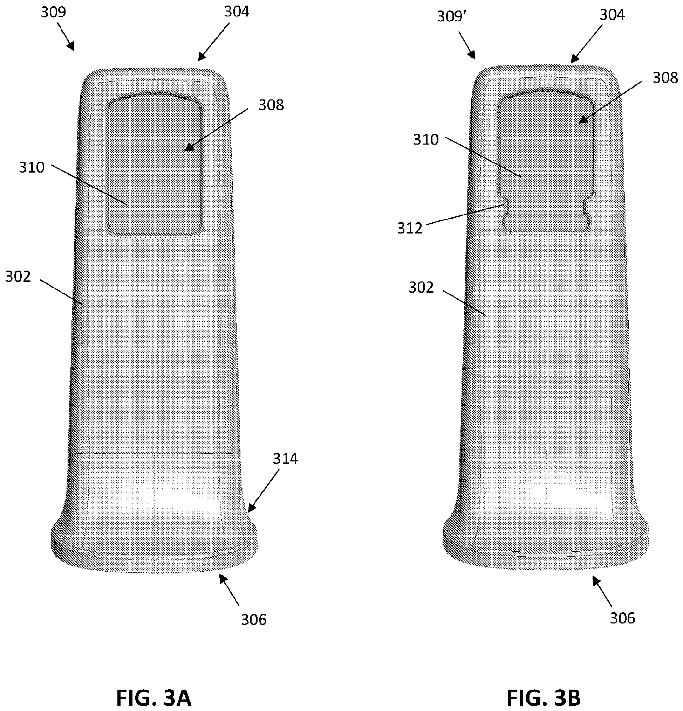

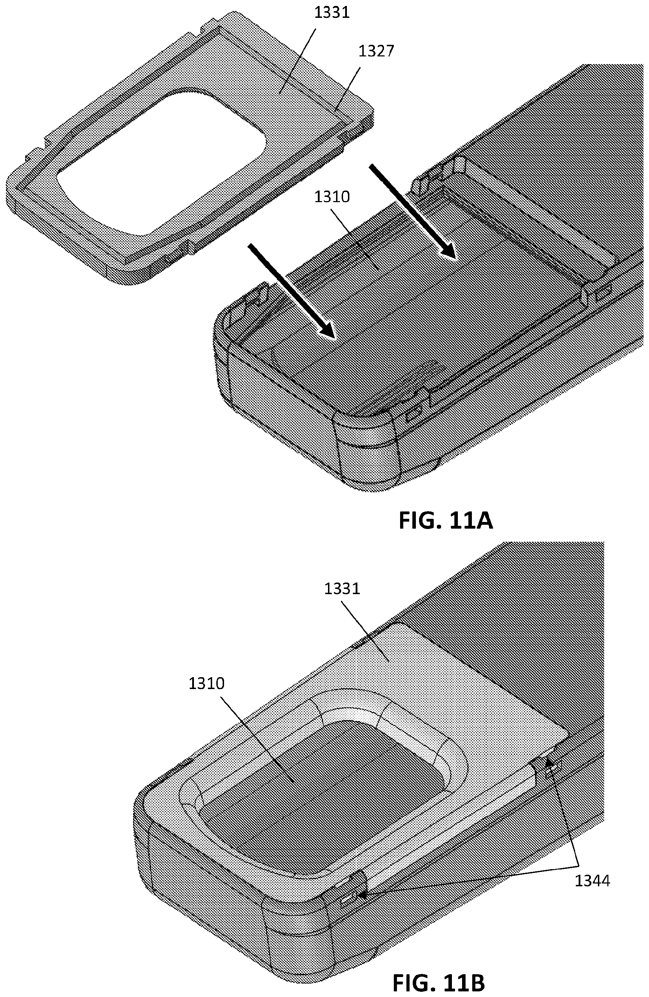

The figures 3A to 10B and 19A and 19B show illustrations of sleeves, some of which including features that may be included in the sleeve of the claimed invention, whereas figures 11A to 18B show embodiments of a sleeve of the claimed invention.

### DETAILED DESCRIPTION

Described herein are protective sleeves for use with intraoral scanners. The protective sleeves may include a body portion, which may be configured to attach to a hand-held wand portion of an intraoral scanner, and a window portion through which the intraoral scanner may transmit light and/or receive images. The window portion may be covered with a transparent cover (e.g., a glass cover, a polymeric cover, etc.). In some examples the protective sleeves ("sleeves") for intraoral scanners described herein may include a recessed lip (e.g., rim, ridge, e.g.) on an outer surface of the sleeve body around a window region in which a transparent cover may be seated. The rim or ridge may be recessed from the outer surface so that the transparent cover may be applied from the outer surface and may therefore be flush with or sit slightly proud of the outer surface of the sleeve body.

In some examples, the sleeve may include an internal guiding structure (e.g., a projection, ramp, slide, etc.) that may guide or direct and/or secure a wand of an intraoral scanner within the lumen of the sleeve so that the imaging region of the wand is aligned with the transparent window, but without scratching or otherwise damaging the transparent window from within the sleeve as the wand is inserted into the sleeve. The internal guiding structure (e.g., ramp) may be separated from sleeve body and may be attached permanently (e.g., by an adhesive, weld, etc.) or temporarily to the sleeve body. One or more internal guiding structures may be included. The one or more internal guiding structures (e.g., ramps) may be positioned and/or shaped so as to guide the wand into the sleeve. Any of the sleeves described herein may be configured for injection molding to form the sleeve as an integral (single body) piece. Also described herein are methods of inserting a scanner (e.g., wand) of an intraoral scanner into a sleeve using the projection (e.g., ramp) to guide the wand into position. The internal guiding structures described herein may alternatively and equivalently be referred to as internal guides or internal guide projections.

Any of the sleeves described herein may include a sleeve extension extending from the rigid or semi-rigid body of the sleeve at the distal end opening so that it may cover more of the intraoral scanner and/or handle, cord, etc. of the intraoral scanner. The sleeve extension may be more flexible than the sleeve. In some examples the material of the sleeve may be formed of the same material as the more flexible sleeve extension.

Any of the sleeves described herein may be configured so that the sleeve may be easily assembled, including in particular attaching or coupling the transparent cover of the sleeve into the sleeve. For example, the sleeve may be configured so that the transparent cover may be added to the sleeve from outside of the sleeve rather than through the inner lumen of the sleeve. In some examples the sleeve may include a sealing frame that may be attached or otherwise coupled to an outside of the sleeve so that the transparent cover is sandwiched between a peripheral region of the window opening and a window opening of the sealing frame. Any of the sleeves described herein may include a seal, which in some examples may be formed of an adhesive sealing material, such as a polymerizing liquid adhesive sealing material that may cure to form the seal. Any of the sleeves described herein may include a channel for holding and/or guiding the seal material around the window. For example, a sealing frame may include an adhesive channel.

The sealing frames for the sleeves described herein may be configured to mate with a seating region in the body of the sleeve. In some examples the seating region may also be configured to secure the transparent cover between the sealing frame and the seating region, including in compression. In some examples the sealing frame may be coupled (e.g., attached) to the body of the sleeve (including into the seating region) by one or more attachments. The one or more attachments may be snaps (including snaps formed integrally into the body of the sleeve and/or the sealing frame), stays, screws, etc.

In some examples the sleeve includes one or more attachments (e.g., snaps, friction fits, etc.) that may be integrally formed into the body, e.g., around the periphery of the window opening through the body. The attachments may be configured to secure the transparent cover to the sleeve without requiring an additional sealing cover. The sleeve window region may include a seating region that is configured to mate with the transparent cover with a high degree of tolerance, such as with a 1 mm or less gap (a gap of between about 0.9 mm and 0.05 mm, a gap of between 0.7 mm and 0.1 mm, a gap or between about 0.5 mm and 0.12 mm, a gap of between about 0.25 mm and about 0.15 mm, a gap of between about 0.22 mm and about 0.17 mm, etc.).

In examples having one or more attachments (e.g., snaps) on the periphery of the sleeve window, the attachments may secure the transparent cover in the seating region of the sleeve. As mentioned, any of these sleeves may also include a seal (e.g., an adhesive seal) around the sleeve window, sealing the transparent cover over the window opening of the sleeve. In some examples an attachment is not included. In any of these examples the transparent cover may be secured to the sleeve body by an ultrasonic weld. As described herein are methods of assembling any of these sleeves, including methods of assembling a sleeve and securing the transparent cover to the sleeve.

Also described herein are sleeves for intraoral scanners in which the window through the sleeve body (at the distal end region of the sleeve) includes a seating region into which the transparent cover is secured. In some examples the transparent window is secured by an ultrasonic weld. As mentioned, any of these sleeves may also include a sealing material around the periphery of the window to seal the transparent cover to the perimeter of the window.

In some examples the body of the sleeve may be configured as a clamshell structure that includes a hinge region (e.g., a living hinge) so that two halves of the sleeve may be folded together (along the hinge region) and secured together to form the sleeve having an inner hollow region (e.g., lumen) into which the wand of an intraoral scanner may be inserted and aligned for imaging through the transparent cover of the sleeve. The living hinge may be on a side of the sleeve, including on the distal end of the assembled sleeve device or it may be located on a side of the assembled sleeve. In assembling a sleeve having a living hinge region, the sleeve may be formed integrally (e.g., by injection molding, etc., as described for other types of sleeves, and may be folded back onto itself to form the assembled sleeve. In some examples, prior to folding back onto itself, the transparent window may be inserted a seating region and sealed into position. Thus, in some examples the transparent window may be inserted from the (future) inside of the sleeve. Once the transparent window is attached (and in some examples, sealed to the body of the sleeve, the sleeve body may be assembled by folding the two conjoined halves together. In some examples, a sealing material (e.g., adhesive) may be applied to secure the two halves together. In some examples the two halves may be secured together by an attachment (e.g., snap, hook, etc.). The two halves of the sleeve may be assembled together and sealed together by a laser and/or ultrasonic weld or welds along the joining region of the two halves.

A protective sleeve may be configured as a rigid, semi-rigid or compliant body that may mate with a hand-held wand portion of an intraoral scanner. The body may be configured to extend over the wand; the protective sleeve may form a barrier against the transmission of contamination such as bacteria, viruses, and like. The body may be configured to act specifically as a barrier to saliva, mucus and other biological fluids. In some variations the protective sleeve, including the body of the protective sleeve, may be formed at least in part from a polymeric material, such as a silicone, latex or other polymer. The body of the sleeve may extend over the wand and/or all or some of a cord or cable. In some examples the sleeve, as mentioned above, may include an extension region for extending over handle, cord and/or cable. For example, the sleeve, including the window and body of the sleeve (also including any extension of the sleeve) may be formed of a flexible barrier material (e.g., a plastic or other polymeric material) that may provide a fluid and/or pathogen barrier.

The protective sleeve may include a window portion that is configured to align with a corresponding window on the wand to transmit light and/or other information for forming images of the patient's dental cavity. The window region may be sized and/or shaped to match or refine the imaging window of the wand. As will be described in some variations, below, the sleeve window may be sized and/or shaped so that at least a portion of the sleeve window projects or extends at least partially into the field of view of the imaging window of the wand, in order to aid in authentication and/or confirmation that the sleeve is applied, and/or is applied correctly. The sleeve window ("transparent cover") may be formed of a transparent material, and in particular may be formed of a material that is transparent to the one or more wavelength(s) used by the intraoral scanner for imaging the patient's dentition. For example, the transparent cover may be formed of an optically clear material, or a material that is transparent in the optical wavelengths and/or the fluorescent wavelength(s) being used and/or the near-infrared wavelengths. The sleeve may be formed of a material that is rigid or semi-rigid and may be a polymeric material, e.g., polycarbonate, polymethyl methacrylate (PMMA), polyethylene terephthalate (PET), amorphous copolyesters, polyvinyl chloride (PVC), liquid silicone rubber (LSR), cyclic olefin copolymers, polyethylene (PE), ionomer resin, transparent polypropylene (PP), fluorinated ethylene propylene (FEP), methyl methacrylate-acrylonitrile-butadiene-styrene (MABS, e.g., transparent ABS), polystyrene (general purpose - GPPS), styrene methyl methacrylate (SMMA), etc. The transparent cover may likewise be formed of any of these materials, and/or may be formed of glass. One or more material (including layers of materials) may be used. The sleeve window may be sealed to the body portion to perfect the barrier against biological contamination. In some variations, all or a portion of the sleeve may also be formed of the same material as the window. In some variations the sleeve may be formed of a material that is different from the window.

In general, the protective sleeve may mate with and engage the intraoral scanner. For example, the protective sleeve may be configured to cover a hand-held wand of an intraoral scanner. In some variations the protective sleeve extends over the end of the hand-held wand so that the window of the protective sleeve aligns with the imaging window of the wand. The body of the protective sleeve may extend over the hand-held wand and in some variations down the body of the wand some distance (e.g., 6 inches or more, 8 inches or more, 12 inches or more, 16 inches or more, 2 feet or more, 3 feet or more, 4 feet or more, 5 feet or more, etc.).

In some variations the protective sleeve may also be textured for gripping (e.g. by a user's hand) securely when operating the intraoral scanner. The protective sleeve may also include one or more ridges, bumps, channels, textures, etc., to assist in gripping.

A protective sleeve may comprise a housing configured to fit over a portion of an intraoral scanning device and protect the intraoral scanning device from an external environment. The intraoral scanning device (e.g., wand) may comprise a first aperture (e.g., opening) for transmission of optical signals, and the sleeve may include a second aperture (the sleeve window which is typically covered by a transparent cover) for transmission of the optical signals. The second aperture may be aligned with the first aperture when the wand is inserted into the sleeve housing. The protective sleeve may be configured so that when the wand is inserted into the sleeve, the transparent cover is aligned with a defogging element of the wand of the intraoral scanner. For example, an external surface of the transparent cover may receive heat generated by a defogging element to prevent fogging of the transparent element. In some examples the transparent cover may be adapted for defogging (e.g., including a coating of an anti-fogging/defogging coating, thermally conductive coating(s), etc.).

The sleeves described herein may be referred to as protective sleeves. The sleeves may include additional materials and components, including lighting (e.g., one or more LEDs), sensors, circuitry, or the like, which may be embedded and/or held within the protective sleeves.

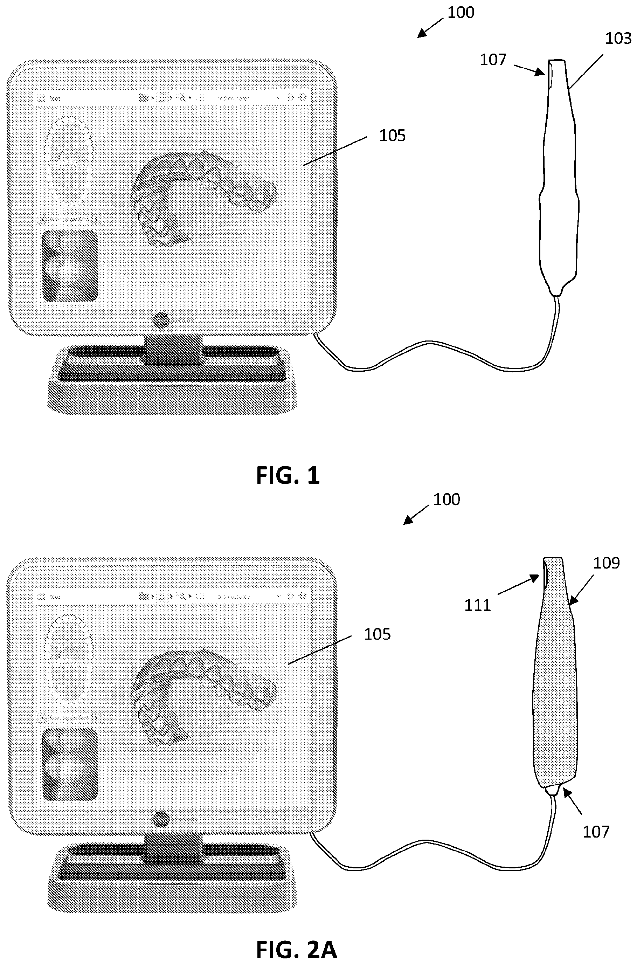

FIG. 1 illustrates one example of an intraoral scanner as described herein, including a hand-held scanner (e.g., wand) 103. One example of an intraoral scanner 100 is the iTero^{®} intraoral digital scanner manufactured by Align Technology, Inc. In general, an intraoral scanner may include a hand-held wand 103, which may include an optical system (including projection/imaging optics) comprising one or more lenses and having an optical axis. The apparatus may also include illumination optics. The apparatus can comprise an axial scanner (e.g., a depth scanning module) that is configured to move the projection/imaging optics system along the optical axis. The apparatus may include a beam splitter configured to transmit light from the light source (after passing through the pattern) to the object and reflect light returning from the object onto an imaging sensor. Thus, the apparatus may include an image sensor configured to receive light returning from the object (via the projection/imaging optics) through the beam splitter. The apparatus can be configured for 3D scanning to at least a portion of the object, for example, intraoral dental 3D scanning for all derivatives of dental restorative and orthodontics indications. The apparatuses for confocal scanning disclosed here can include a confocal illuminator. The optical system may include a projection/imaging system or subsystem including projection optics and imaging optics. For example, the projection optics and the imaging optics can be configured to share the same optical elements (lenses) and the same optical path. The apparatus can comprise the depth scanning module, which comprise a compact linear actuator, for example, a voice coil motor (VCM). The scanner can comprise a front tip, which can include a 45-degree (e.g., back-heated defogging) fold mirror.

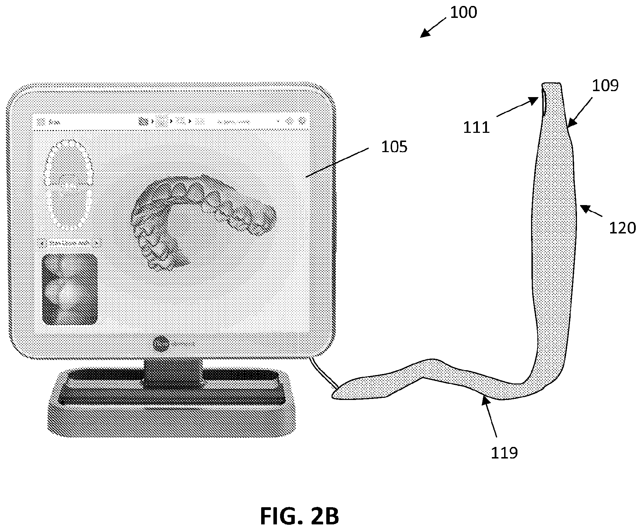

The scanner may include one or more processors and may include on ore more illumination sources (LEDs, lasers, etc.). In FIG. 1, the hand-held wand 103 also include a window 107 providing optical access into the scanner. The window may include an optically transparent cover. The scanner may be wireless or wired to additional components of the system, including one or more additional processors. The scanner may generally illuminate and/or image in the visible spectrum, in the infrared or near-infrared spectrum, in the florescence spectrum, etc. A display 105 may also be included as part of the system. The intraoral scanner shown in FIG. 1 may be used with one or more sleeves, as shown in FIGS. 2 and 3.

In FIG. 2A, the intraoral scanner 100 includes a protective sleeve 109 that is placed over the hand-held wand 103, so that a sleeve window 111 is aligned with the window in the scanner 107 (not visible in FIG. 2A). FIG. 2B shows an alternative variation in which the sleeve 109 is longer, extending over the wand and connecting cable. Thus, the sleeve shown in FIG. 2B includes a sleeve extension 119 at the proximal end; the distal end of the sleeve (the sleeve body 120) may be formed to have a more rigid or semi-rigid body, while the more proximal end extending from this sleeve body may be more flexible. In some examples the sleeve body is formed from the same material (e.g., poly a polyethylene material) as the sleeve extension; the two regions may be welded together (e.g., by laser welding, ultrasound welding, etc.). The difference in rigidity of these regions may depend on the thickness of the material.

FIGS. 3A-3B illustrate two examples of protective sleeves 309, 309' as described herein. FIG. 3A shows the sleeve body 302, which may be an elongate and hollow body having a distal end 304 and a proximal end 306, wherein the very distal end (of the distal end region) is tapered and closed, and the proximal end is open to receive an intraoral scanner wand. The sleeve also includes a window opening 308 that is covered by a transparent cover 310. The base (the proximal end) of the sleeve body in this example is flared outwards 314.

FIG. 3B shows another example of a sleeve that is very similar to that shown in FIG 3A but includes a pair of protruding regions 312 at the window periphery, which project slightly into the field of view of a wand that is inserted into the inner hollow body of the sleeve.

Thus, any of the sleeves may include a notch, protrusion (e.g., bumps, projections, etc.) on the sleeve window, such as the periphery of the sleeve window, that may be within the field of view of the intraoral scanner when the sleeve is attached and may be detected by the intraoral scanner. In FIG. 3B the protrusion is formed of the window of the sleeve itself in an area that is exposed to the camera

As mentioned, the sleeves described herein may be fabricated as a single unitary device, to which (in some examples) the transparent cover may be added. Alternatively, in some examples the material of the entire sleeve may be transparent, and the transparent cover may be integrated (and integrally formed) with the rest of the body of the sleeve. In some examples, the body of the sleeve is formed of a polymeric material such as PE (Polyethylene) or materials that include PE with other materials. For example, the sleeve may be formed of a mixture of HDPE and LDPE (e.g., between 60-95% HDPE and between 5-40% LDPE). The PE may be laser welded as described herein to secure, e.g., the extension region.

As mentioned above, any of the sleeves described herein may include one or more internal (within the hollow cavity of the sleeve) guiding structures, such as ramps, to guide and/or lock (e.g., releasably lock) the wand in position so that it is aligned and/or secured within the sleeve. Any of these internal guiding structures may also be retainers that retain (releasably retain) the wand within the sleeve and prevent it from moving substantially within the sleeve, which may miss-align the wand imaging optics and the transparent cover (or window) of the sleeve.

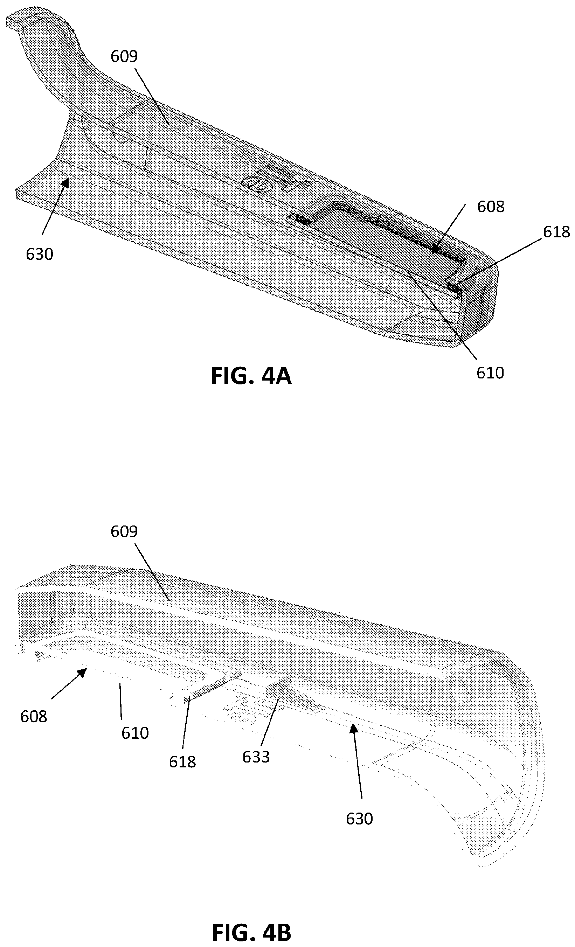

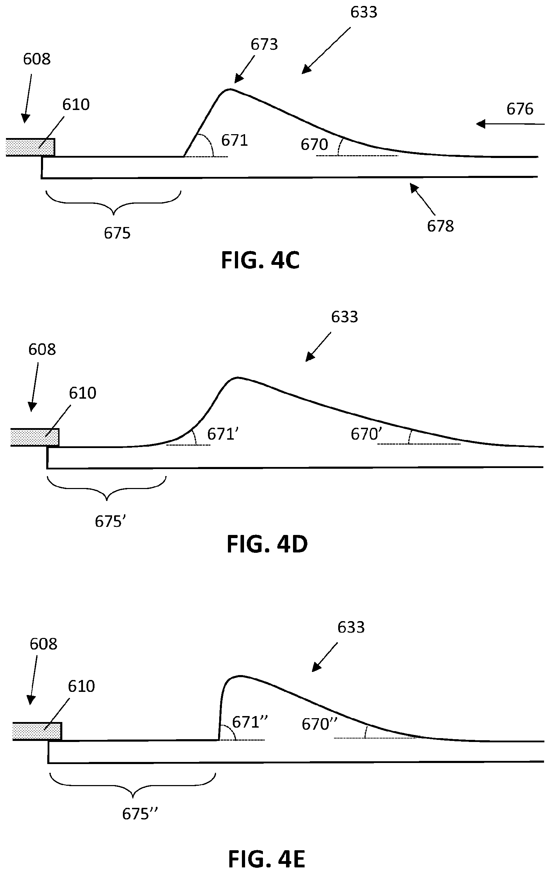

In some examples the internal guiding structure is formed integrally (as a single piece, e.g., as by injection molding) with the body of the sleeve. Alternatively, in some examples the internal guide structure (e.g., ramp) is a separate element that is coupled to the inner wall of the sleeve body. For example, the internal guiding structure may be configured as a ramp that is positioned just proximal (e.g., within about 1 cm, within about 2 cm, within about 3 cm, within about 4 cm, within about 5 cm, within about 6 cm, within about 7 cm, within about 8 cm, within about 9 cm, within about 10 cm, etc.) to the window opening region of the sleeve, on the internal surface of the sleeve. In some examples the internal guiding structure is a ramp formed of a plurality of triangular ribs or wings that extend from the inside of the sleeve body. For example, FIGS. 4A and 4B illustrate cross-sections of an example of a sleeve similar to those shown in FIGS. 3A-3B. FIG. 4B shows the inside 630 of the sleeve 609 including an internal guiding structure 633 configured as a ramp or slide. In this example, only a single ramp/slide is shown; additional ramps/slides may be positioned adjacent to the window (in the half of the sleeve not shown in FIG. 4B for example).

The internal guiding structure (e.g., ramp or slide) may generally have a surface that extends from the inner wall of the sleeve up above the height of the window region relative to the inter wall, which may guide the wand and prevent it from scraping or scratching the inside of the window region 608 of the sleeve (e.g., the transparent cover in variations including a transparent cover).

FIGS. 4C-4E illustrate examples of internal guiding structure (e.g., ramps) that may be used, shown in cross-section. For example, in FIG. 4C the internal guiding structure has an approximately triangular cross-section for guiding a wand into the hollow distal end of the sleeve. As the wand is slid distally 676, it may be guided by the ramp distal face to side up, away from the edge of the window 608. In this example the distal face has an angle 670, 670', 670" relative to the wall of the cover from which it is formed (and extends into the inner hollow of the sleeve) that may be between about 10 degrees and about 50 degrees (e.g., between about 10 degrees and about 45 degrees, between about 10 degrees and 40 degrees, between about 15 degrees and 50 degrees, etc.). The ramp may be offset a distance 675 from the window opening 608 and thus the transparent cover 610. The opposite side of the ramp may also be curved and form a ramp face that may help remove the wand from the sleeve back over the ramp; in some variations this second, proximal-facing ramp may be angled relative to the wall of the cover from which the ramp is formed with a greater angle than the distal-facing ramp. For example the angle 671, 671', 671" of the proximal-facing ramp may be between about 15 and about 90 degrees. Either or both the distal-facing ramp and the proximal-facing ramp may have a continuous (e.g., smooth) curve as shown in FIGS. 4A-4D. In FIG. 4E the proximal-facing ramp is an abrupt transition (having a 90-degree angle). The spacing (offset distance) 675, 675', 675" between the ramp and the window opening may be, e.g., between about 1 mm and about 2 cm (e.g., between 1 mm and 1 cm, between 1 mm and 9 mm, etc.). In some examples the internal guiding structure is not offset from the edge of the window region 608, but may be immediately adjacent to it. In each of the examples of internal guiding structure shown in FIGS. 4C-4E, the internal guiding structure is a ramp having a triangular cross-section in which the top of the ramp is shown with a curved corner 673. This corner may be rounded (as shown) or sharp. In some examples the top is flattened. For example the internal guiding structure may have a trapezoidal shape (which may have sharp or rounded edges). The examples of internal guiding structures shown in FIGS. 4C-4E are also shown with sleeves in which the transparent cover 610 of the window region 608 is shown schematically on the inside of the cover. Alternatively, as will be described in greater detail below, the transparent cover may be on the outside surface 678 or may be flush (e.g., within a seating region) of the window region.

As described above, the internal guiding structure 633 may comprise a plurality of fins, as shown in FIG. 4B. In this example, the internal guiding structure includes two thin fin-like lengths extending in the proximal-to-distal direction within the inside of the sleeve and having a triangular longitudinal cross-section. In some examples the proximal-facing side of each ramp may be connected to a surface that extends between the fins (forming a proximal-facing surface). This proximal-facing surface may be optional; in some examples the internal guiding structure is formed of an arrangement of spaced apart (e.g., by 0.1 mm, 0.2 mm, 0.3 mm, 0.4 mm, 0.5 mm, 0.7 mm, 0.8 mm, 1 mm, 1.1 mm, 1.2 mm, etc., between 0.1 mm and 3 mm, between 0.1 mm and 2 mm, between 0.1 mm and 1.5 mm, between 0.1 mm and 1 mm, etc.) the fins extending from the inner wall of the body of the sleeve. Any number of fins (e.g., 1, 2, 3, 4, 5, 6, 7, 8, etc.) may be used for each internal guiding structure.

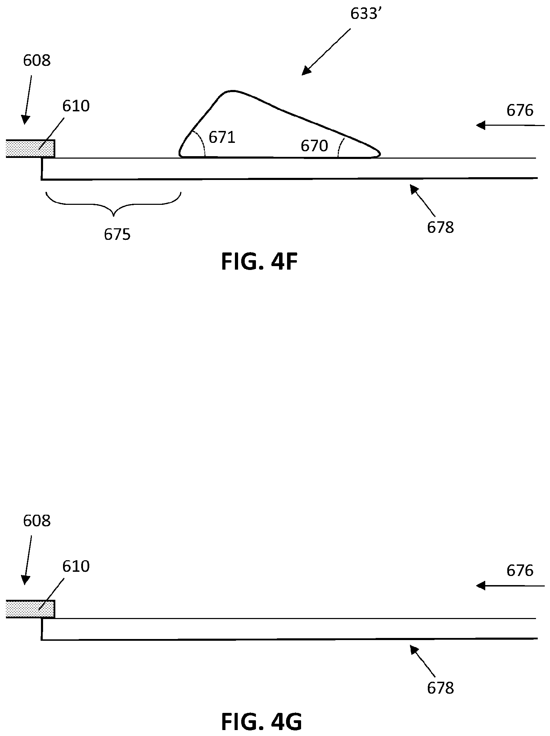

In any of these apparatuses, the ramp may be formed separate (rather than integrally) and attached to an inner surface, as shown in FIG. 4F. In this example the internal guiding structure 633' (e.g., ramp) is a separate element that may be affixed to the inner wall of the sleeve body. In some example, the sleeve does not include a ramp, as shown in FIG. 4G.

The example of the sleeve shown in FIG. 4A includes the transparent cover attached on the inside of the sleeve. In this example, the transparent cover 610 forming a portion of the window region 608 is sealed to the periphery of the window region by a foam (window foam) material. The foam may be, for example, MED 5696R Adhesive foam (e.g., Avery Dennison) or MED 2123R adhesive (Vancive Medical Technologies). The foam may be compressible and may adhesively secure the transparent cover to the body of the sleeve. Some examples of the sleeves described herein do not include foam. In some examples an elastic material may be used instead of foam. The elastic material may be an adhesive that can be applied as a liquid or gel (or paste) but that cures into a solid, forming a seal between the body of the sleeve and the transparent cover. The same or a similar foam material may be used in any of the devices described herein.

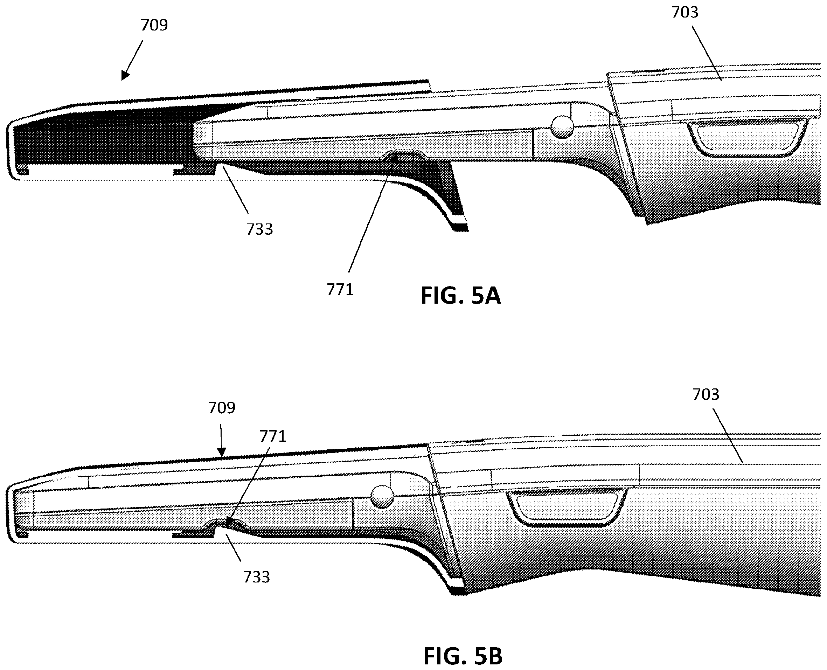

In use, the cover may be applied over the distal end of a wand, for example by sliding the wand into the cover or by sliding the cover over the wand, or some combination. The wand of the intraoral scanner may slide within the hollow inner region of the sleeve and the internal guiding structure (ramp) may deflect the distal tip up and away from the transparent window (e.g., in some examples the transparent cover of the window) which may protect the transparent window and allow the wand to be positioned optimally relative to the sleeve. For example, FIGS. 5A-5B illustrate one method of attaching a cover 709 to a wand of an intraoral scanner 703.

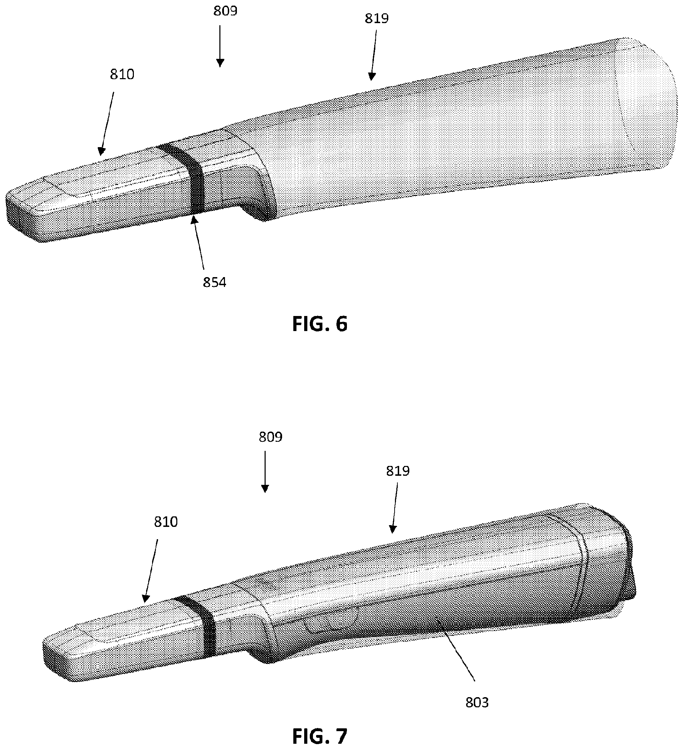

In FIG. 5A, the distal end of the wand is inserted into the distal end opening of the sleeve, and advanced to the internal guiding structure (e.g., ramp or slide 733) The internal guiding structure guides the tip of the wand above the window region and may act as a securement to reliably hold the wand in position, as shown in FIG. 5B. As shown in FIGS. 5A and 5B the wand includes an engagement region 771 for engaging the internal guiding structure. The engagement region in the body of the wand in FIG. 7 is shown as a recessed region or opening that includes surfaces that may engage with the internal guiding structure; once the wand is positioned distally far enough into the sleeve, the internal guiding structure may fit into the engagement region of the wand, allowing the tip of the wand, including the imaging window of the wand, to be aligned with the window region of the sleeve in a fixed position. The engagement region may also engage releasably hold the wand in this position relative to the sleeve until the sleeve is withdrawn proximally. In the example shown in FIG. 7, when the wand is fully extended distally the imaging window of the wand may push down against the window region, which may include a foam material, as described above, which may be compressed. The wand of the intraoral scanner may be disengaged from the internal guiding structure by pulling the wand proximally (and/or the sleeve distally) with sufficient force so that the sidewall of the engagement region slides along the proximal-facing side of the internal guiding structure, driving the wand up, away from the window region of the sleeve, and allowing it to be withdrawn out of the sleeve.

Any of the sleeves described herein may include a sleeve extension portion that extend proximally from the more rigid (or semi-rigid) proximal end, as shown in the examples of FIGS. 6 and 7. In FIG. 6 the sleeve 809 includes a more rigid distal sleeve portion 810 and a more flexible proximal sleeve extension portion 819. The proximal sleeve portion may be attached to the distal sleeve by an adhesive or a weld. In some examples the sleeve extension is welded (e.g., laser welded) 854 to the sleeve (the distal portion of the sleeve). The material forming the sleeve extension may be the same as the material of the distal portion of the sleeve (e.g., polyethylene or a mixture of polyethylene and other polymers or other forms of polyethylene). The sleeve extension may be configured to invert over itself when pulled distally, such as when removing the sleeve from over the wand.

FIG. 7 shows an example of the sleeve of FIG. 6 applied over a wand 803 of an intraoral scanner. The sleeve extension may extend for between 5 inches and 5 feed from the distal sleeve portion (e.g., between 6 inches and 4 feet, etc.). The sleeve extension may be configured to roll up over itself for removal, so as to trap any contamination on the outer surface of the sleeve within the rolled-up portion. Thus, in some examples the sleeve extension be tapered with a wider proximal end than the distal region coupled to the distal (more rigid) sleeve region. In some examples the sleeve extension may be biased (pre-rolled) back over itself. For example, a new and clean sleeve may include a rolled-up sleeve extension that may be rolled out proximally when applied over the wand. The rolled-up sleeve may be rolled so that the more distal portion is rolled within the more proximal portion. Once rolled out the sleeve extension may remain unrolled during use (and in some examples may include a fastener, such as an adhesive and/or tie, to remain unrolled and applied over the wand or cord of the wand) until it is removed. The sleeve extension may therefore be biased to roll back up to the original configuration.

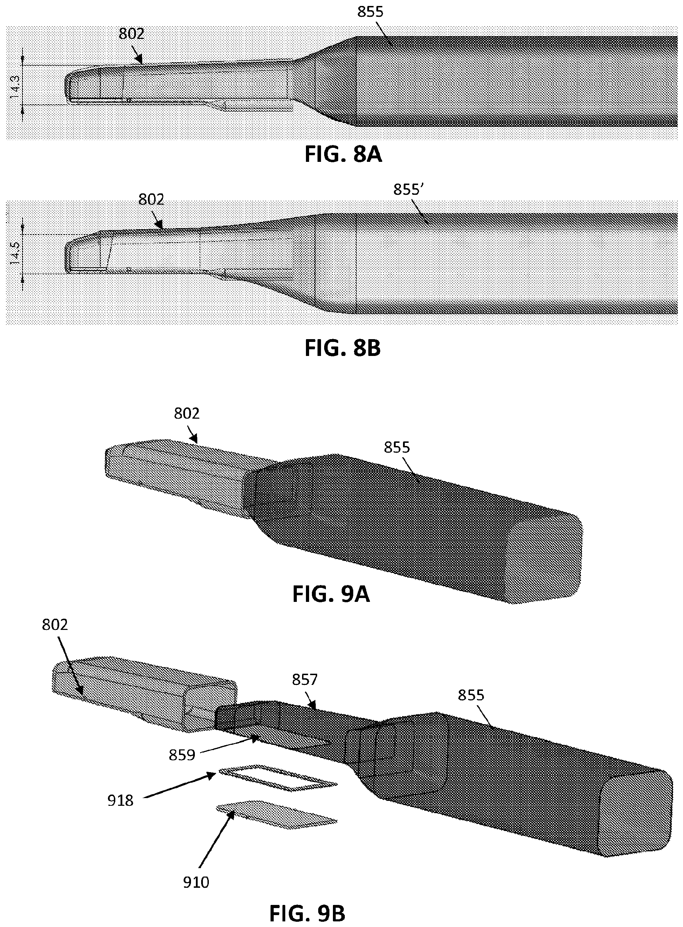

The sleeve extension may be part of and/or affixed to the sleeve body (as shown in FIG. 6 and 7), or it may be inserted into and/or over the sleeve. For example, FIG. 8A shows an example of a sleeve extension configured as an internal sheath bag 855 that is inserted into the more rigid sleeve body 802. The sleeve extension may fit over the distal end and a portion of the wire of the scanner (or wand) of the intraoral scanner. FIGS. 9A-9B show a perspective view and an exploded perspective view of one example of a sleeve such as the one shown in FIG. 8A. As described herein, the sleeve extension may be formed of a thinner, lighter and/or more flexible material and may include a window region 859 that fits over the window region of the more rigid sleeve body when the sleeve extension and sleeve body are coupled with the scanner. The sleeve also includes a transparent cover 910 and a gasket 918 that is configured a foam frame that may be positioned in the window (e.g., in a lip or ridge region of the outer surface of the window) between the transparent cover and the lip region of the sleeve body 802. In some examples the sleeve extension may be secured to the rigid sleeve body, for example by an adhesive (e.g., glue), weld, or the like. The sleeve insert may be affixed to the sleeve body at or around the window 859, and/or at or around the proximal end of the sleeve body. Alternatively, in some examples the bag-like sleeve extension that is inserted into the sleeve body may be held by a friction fit. The sleeve extension (e.g., sleeve bag) 855 in FIGS. 9A and 9B may be a barrier of polyethylene in some examples.

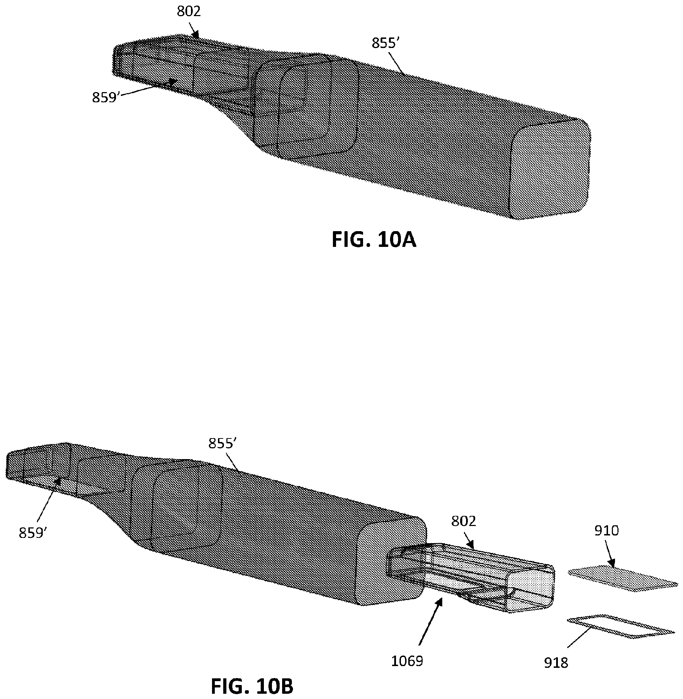

FIG. 8B shows another example of a sleeve including a sleeve body 802 and a sleeve extension 855'. In this example the sleeve extension is configured to fit over the sleeve body. The dimensions shown in FIGS. 8A and 8B are exemplary. IN FIG. 8B and the example shown in FIGS. 10A-10B, the sleeve extension 855' is an external sheath or bag into which the more rigid sleeve body 802 fits into. The external sleeve extension may be used in a manner similar to the internal sleeve extension shown in FIG. 8A; in both examples the sleeve extension may be attached (e.g., welded, glued, etc.) to the more rigid sleeve body 802, or simply secured by a friction fit. In examples in which the sleeve extension is not attached to the sleeve body, the same sleeve body may be re-used with multiple different sleeve extensions.

As shown in FIGS. 10A-10B, the external sleeve extension 855' may include a window region 859' that may be a cut-out region that aligns with the window opening of the sleeve body 802. As shown in the exploded view of FIG. 10B, the transparent cover 910 and gasket (e.g., foam gasket) 918 may seal the transparent cover over the window region 1069 against a lip region of the inner or outer region of the sleeve body.

In some examples the transparent cover may be secured to or against the sleeve body by an adhesive and/or a weld, including in particular an ultrasonic weld. Alternative or additionally, in some examples the transparent cover may be secured to the sleeve body by a sealing frame. The sealing frame may be secured to the body of the sleeve (the rigid or semi-rigid distal portion of the sleeve) by an adhesive and/or by a mechanical fastener such as a snap or the like. For example, FIGS. 11A-11B illustrate another example of a sleeve including a sealing frame 1331 that seals a transparent cover 1310. The window may be placed into the seating region of the sleeve, which is sized and configured to receive the transparent cover as shown. The transparent cover may be easily positioned without requiring force (pressure) to be applied. Once seated in the receiving area (e.g., the seating area or region, such as a lip or rim), an adhesive may be applied to the periphery of the sealing frame 1331 such as within a channel ("adhesive ditch") 1327 and the sealing frame 1331 may be applied over and into the receiving area of the sleeve as shown in FIG. 11B. In FIGS. 11A-11B the receiving area 1331 is recessed so that the sealing frame sits flush with the outer surface of the sleeve when the sealing frame is secured down. The sealing frame may also or alternatively be secured to the rest of the sleeve by one or more snaps 1344. In examples in which a frame is used, the frame may mechanically hold the transparent cover in place until the adhesive is cured, without requiring a fixing jig.

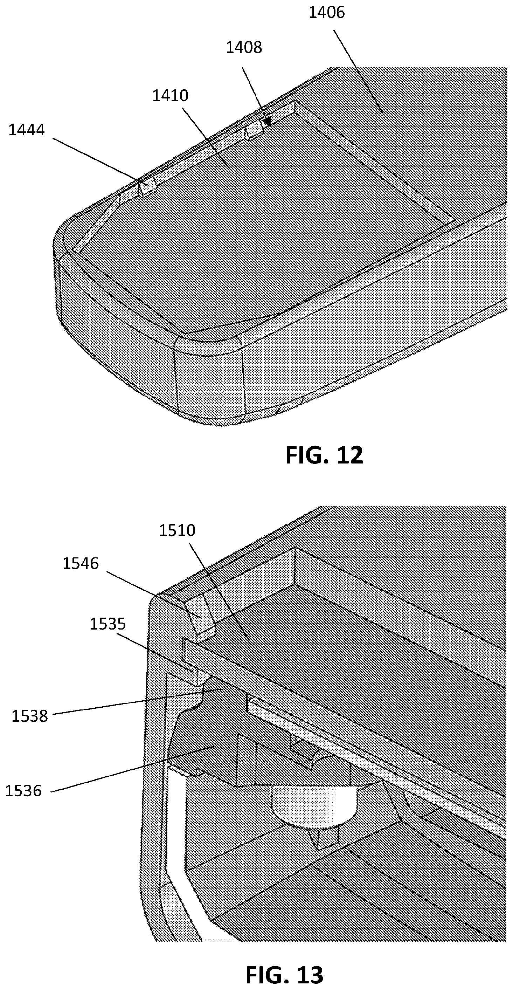

In some examples the sleeve may include a receiving area in the distal region of the body of the sleeve around the window opening of the sleeve that is configured to receive and hold a transparent cover 1410 without requiring a sealing frame. In some examples the transparent window is secured by an adhesive applied around the window opening 1408 of the body of the sleeve 1406 either before or after the transparent window has been applied. For example, FIG. 12 shows one variations of a sleeve in which a seating region is included at the distal end. The window region includes a peripheral seating area in which one or more snaps 1444 are included. The snaps are formed from the sleeve body (as a unitary portion) and may include a ramped overhang portion 1546 and an undercut region; the transparent cover 1510 edge may slide against the ramped overhang portion and displace (e.g., bend) it out of the way (or may itself bend out of the way) so that it may seat into the seating region 1535, as shown in FIG. 13. An adhesive may be applied around the periphery of the window opening to seal the transparent cover within the window opening (not shown). FIG. 13 also shows a section through the sleeve while the sleeve is being worn over a wand of an intraoral scanner 1536. In this example the wand also includes one or more spacing projection regions 1538 that may contact the transparent cover 1510.

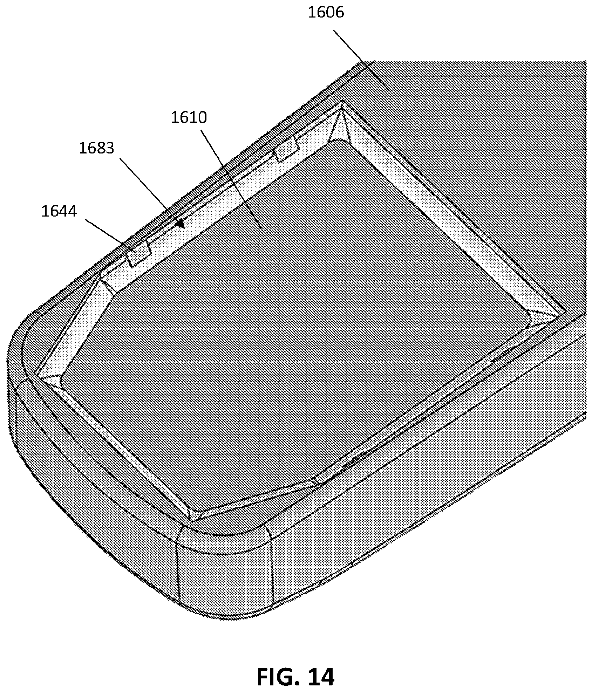

The adhesive may be added at the end, and from the outside, which may reduce the risk of contamination of any of the optics (e.g., windows) and may be easier and less expensive. For example, FIG. 14 illustrates the sleeve of FIG. 13 with an adhesive seal 1683 (which may be fully cured) shown. In FIG. 14 the transparent cover (window 1610) is held in place by the snaps 1644 formed integrally into the body along with the seating region of the sleeve 1606. In some examples one of the sides of the window opening may include a shelf and an overhang rather than a snap; the overhang may secure the transparent cover on one side and one or more snaps may be on the opposite side of the window opening and shelf (seating area).

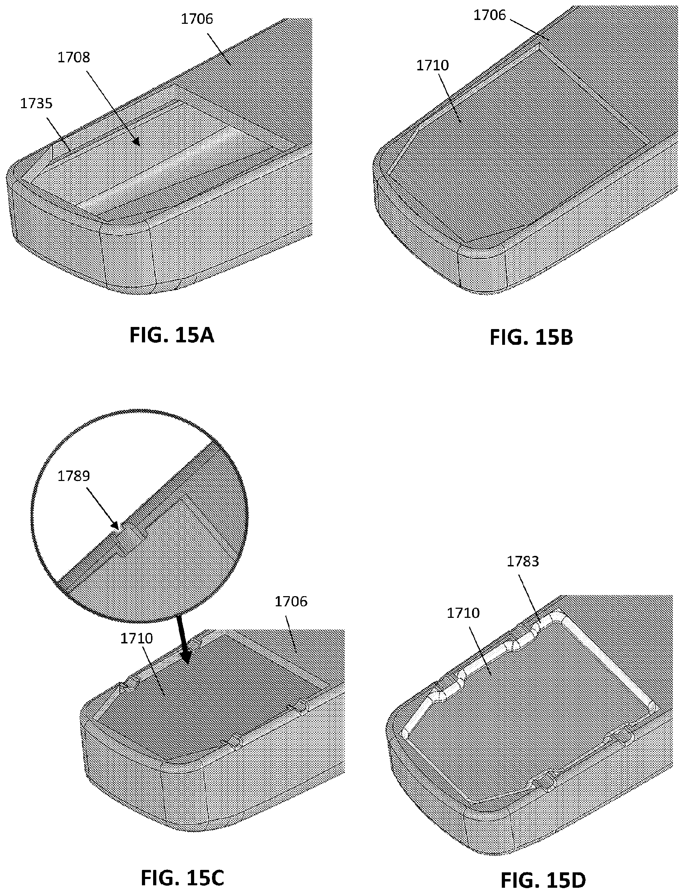

Also described herein are examples in which rather than (or in addition to) snaps, the transparent cover may be secured to the sleeve within a seating area 1735 by heat pressing to deform a region of the window perimeter of the sleeve, holding the transparent cover in place. FIGS. 15A-15D illustrate one example of a sleeve in which the transparent cover is heat-pressed (heat staked) in position. In FIG. 15A, the body of the sleeve 1706 includes a window opening 1708 with a seating area 1735 having a lip around the perimeter of the window opening. A transparent cover that fits the seating area (in size and shape) may be positioned onto the seating area from outside of the sleeve, as shown in FIG. 15B. FIG. 15C shows that multiple regions of the periphery may then be deformed to secure the transparent cover within the seating area, by (in one example) heat pressing. A heat may be applied at one or more points to melt and deform the polymeric peripheral region so that it forms a heat stake 1789 that extends over and locks the transparent cover in position, as shown. An adhesive sealing material 1783 may then be applied to seal the edges of the transparent window 1710 to the sleeve, as shown in FIG. 15D. Similar to the snap-fit configuration shown in FIGS. 12-14, this sleeve example may also be formed as a single injected part implementation into which the transparent window may be attached.

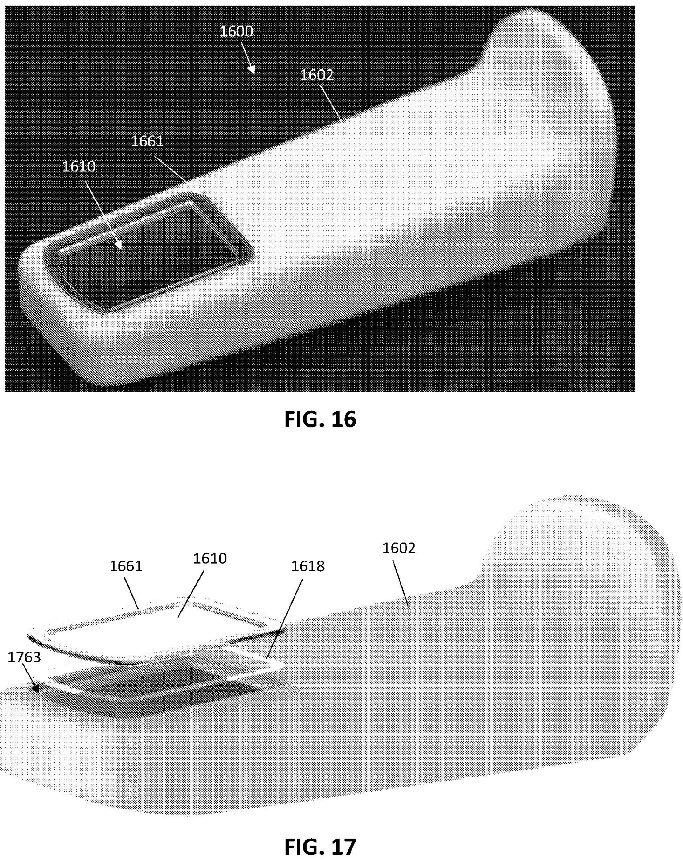

In some examples the sleeves described herein include externally mounted transparent covers, such as the example sleeve 1600 shown in FIGS. 16 and 17. This sleeve may include a sleeve extension (not shown) and a sleeve body 1602. The externally mounted transparent cover 1610 is held in a lip region 1763 that is cut into the outer surface of the sleeve body 1602. The lip region extends into the sleeve body and forms a rim into which the transparent cover 1610 may sit. In some examples a gasket (e.g., foam gasket, rubber gasket, silicone gasket, etc.) 1618 may be positioned between the rim region and the transparent cover, and/or over the edge of the transparent cover. A seal 1661 may be secured over the edge of the transparent cover and with the sleeve body. The seal may be a frame. The seal may be fluid tight. In some examples the seal is formed by a cement or adhesive; in some examples the seal is formed by welding (e.g., laser welding, ultrasonic welding, etc.).

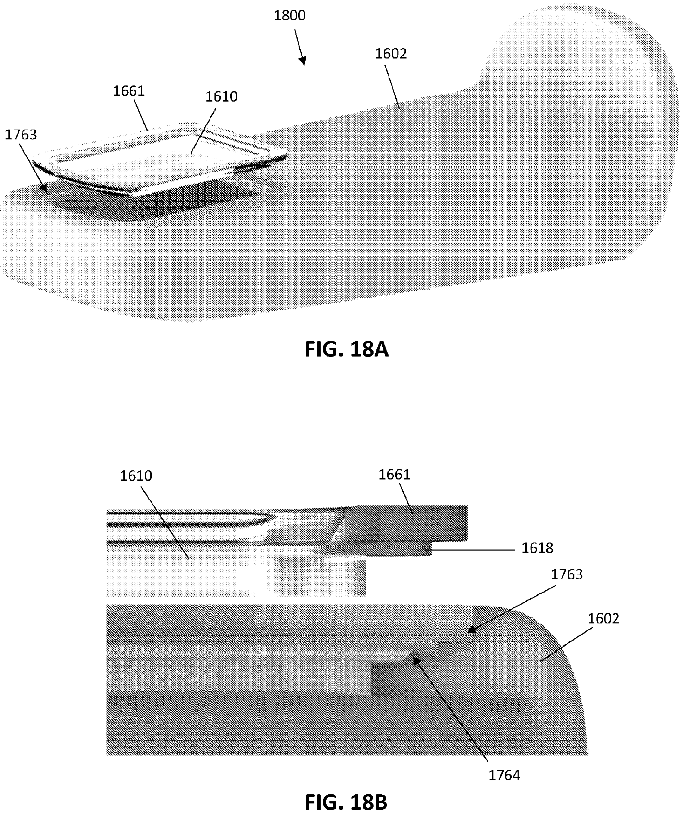

FIGS. 18A and 18B show an example of a sleeve including an externally mounted transparent cover 1610 similar to that described above. In FIG. 18A the sleeve 1800 include a relatively rigid or semi-rigid body 1602 having an outer surface and an inner surface. The scanner (e.g., wand) of the intraoral scanner may fit into the opening or cavity of the sleeve body. A window into the sleeve body is formed as a cut-out region having an annular lip or rim 1763 that is cut into the outer surface of the sleeve body from the external surface. As shown in the profile of FIG. 18B, the rim or ridge may include a lip formed and extending from the inner side so that the opening on the external surface side is slightly larger than the opening through the sleeve body on the internal surface. The lip may include one or more (e.g., a continuous) protrusion 1764 that may seal against a gasket 1618 between the transparent window 1610 and the sleeve body. The gasket may act as a seal. As shown in FIG. 18B the gasket 1618 may be adjacent to the transparent cover 1610 and may be sandwiched between the sleeve cover 1602 and the outer seal 1661.

In general, the sleeves descried herein may be coated with a material, such as an antireflective coating as will be described in greater detail below. In some examples both sides of the transparent cover 1610 may be coated with a double sided AR coating, which may improve scanner signal. The thickness of the transparent cover as well as the distance between the transparent cover and the window of the scanner may be precisely controlled. In addition, the ultrasonic welding may be used as indicated above to increase the reliability of the seals for increased safety. As mentioned above, any appropriate material may be used, including, for example, polyethylene (PE) and/or polycarbonate (PC).

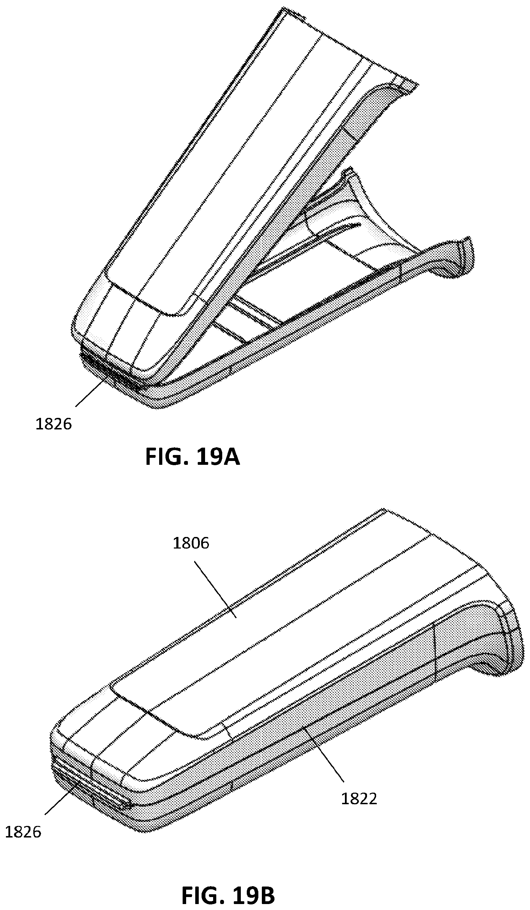

In some examples the transparent cover forming the window may be applied internally without having to be applied through the inside of the sleeve. For example, FIGS. 19A and 19B illustrate an example of a sleeve having a body that is formed as a clamshell including a living hinge region that may be molded or otherwise formed as a single piece (e.g., by injection molding) and may be closed along the living hinge to form the hollow sleeve (e.g., the distal, rigid/semi-rigid sleeve portion). FIG. 19A shows the body prior to closing (or during closing) while FIG. 19B shows the sleeve in the closed configuration. The edges 1822 of the sleeve 1806 where the two halves of the sleeve come together may be sealed, using an adhesive or by welding (e.g., laser welding) of the material. In this example the living hinge 1826 is on a distal end; in some examples the living hinge may be on a side and the top region may be sealed. Thus, the sleeves described herein may include an integral folding feature on a side edge or a distal edge.

In general, any of the sleeves described herein may include features shown in any of these examples. Other sleeve designs may also incorporate all or some of these features. For example, in some cases the sleeve may be fabricated as a single piece, including the transparent window; for example, the entire sleeve (e.g., the sleeve distal end in regions including a sleeve extension) may be transparent. For example a transparent sleeve including an integral transparent cover may include the internal guiding structure (e.g., ramp) as described herein.

The sleeves described herein may be configured to withstand pressure of at least about 50 kPa, such as at least about 60 kPa, at least about 70 kPa, at least about 80 kPa, at least about 90 kPa, at least about 100 kPa, etc. In any of these sleeves the sleeve may be configured, e.g., by the position of the seating area for the window, so that there is a minimum separation between the sleeve window (transparent cover) and the imaging window of the wand. For example, the minimum clearance between the sleeve window and the imaging window may be about 0.1 mm, about 0.2 mm, about 0.3 mm, etc. This minimum distance may also be maintained in part based on a spacing projection adjacent to the imaging window of the wand, as described above.

Any of the sleeves described herein may include a flavor. For example, the sleeve (particularly the distal end region) may be coated or may incorporate a flavorant. Any appropriate flavorant may be used. Flavorants may be coated by dipping, spraying or the like.

As described above, in any of the variations described herein the systems and/or sleeves described herein may be configured for single, one-time (e.g., one continuous session) or a limited duration of time use.

### ANTIREFLECTIVE MATERIALS

Any of the apparatuses described herein may include one or more antireflective materials, and in particular an antireflective material that is appropriate for the visible spectrum (e.g., 400 nm to about 850 nm) and/or near-IR range of frequencies of light. For example, any of these apparatuses may include an antireflective material on the transparent cover of the window of the removable barrier (sleeve) devices. The antireflective material may be formed of a nanostructured material (e.g., "moth eye" material). This material may be applied as a laminated layer that is applied to the device (e.g., to the transparent cover). The material may be a hydrophobic material that is applied to the sleeve/removable protective cover of the device or, in particular, to the removable transparent cover a barrier that is applied over the intraoral scanner.

Reflections, such as specular reflections, are particularly problematic in intraoral scanners that have a high level of back reflections off of the transparent cover to the camera within the intraoral scanner, e.g., when the transparent cover for the intraoral scanner is located close to the focal point of the sensor (e.g., within a few cm), and/or where internal polarizers may not be used, e.g., because the polarization depth is insufficient.

Thus, described herein are methods and apparatuses (e.g., devices) in which an anti-reflective layer is included (e.g., on the intraoral scanner window and/or the transparent cover of the removable barrier for the intraoral scanner). An antireflective layer may be a structure, e.g., coating, film or lamination, that is applied to the window of the intraoral scanner window and/or the transparent cover of the removable barrier. In some variations the anti-reflective structure is a nanostructure that is configured as an anti-reflective material configured to reduce or eliminate reflections, in particular reflections in the near-IR range of light. The anti-reflective material may be a nanostructure arrays (NSAs); these materials may include silicon and non-silicon materials (e.g., nanoporous SiO₂) and may have a structure that is configured to operate as an anti-reflective material in the nanostructured range.

The anti-reflective structure may be a hydrophobic structure forming a nanostructured film that reduces or eliminates reflections. A typical "moth eye" anti-reflective structure may have a hexagonal pattern of nanoscale bumps that are smaller than the wavelength of the light applied (e.g., in this case, near-IR light). In some examples, anti-reflective structures that may be used include stickers of material that may be laminated to a transparent cover to prevent or reduce reflection.

An intraoral 3D scanners may include a transparent cover with an anti-reflective coating by layer deposition in vacuum chamber. In particular, described herein are removable protective covers/sleeves for intraoral scanners having a transparent cover with a hydrophobic (moth eye) anti-reflective structure. These apparatuses (e.g., devices) and methods, e.g., methods of implementing the antireflective structures as described herein may include direct lamination of a material (e.g., as a "sticker") on a molded sleeve/removable barrier for the intraoral scanner. The anti-reflective material ("sticker") may be laminated on a glass/polymer transparent cover, e.g., by applying the sticker that has the nanoscale structure forming the moth-eye antireflective coating already formed, and the transparent cover assembly including the laminated antireflective sticker may be combined with the body of the sleeve/protective cover, as described above. In general, the moth eye structure may be added to the transparent cover as part of an injection molding process of the transparent cover or of the whole sleeve/protective cover.

In general, a nanostructured (e.g., "moth eye") antireflective structures described herein may be formed and applied to the transparent cover as a sticker or lamination; alternatively or additionally, these nanostructured antireflective surfaces may be formed as a coating or layer directly on the transparent cover (e.g., without using a "sticker" configuration, which may include an additional adhesive material). In some variations the antireflective structures described herein may be formed directly on the transparent cover. For example, a nanostructure forming an AR layer or coating may be formed during the injection process of forming the sleeve. For example, a mold for forming the sleeve and/or the transparent cover (or in some cases, both) may include an insert stamp with nano structures. Either or both the internal and external surfaces of the transparent cover may include the AR layer.

Any device or apparatus may include an anti-reflective layer or coating on the transparent cover that is formed by one or more stickers. For example, one or more stickers including a nanostructure may be laminated on the optical area of the transparent cover. In some examples only one side (e.g., the external side) may include the coating/sticker (e.g., antireflective, AR). The anti-reflective laminated "stickers" described herein may be particularly advantageous. For example, these structures may be formed at relatively low cost (e.g., as compared to class anti-reflective coatings), and can be added to glass or polymeric transparent covers for removable protective covers/sleeves for intraoral scanners. These structures may achieve similar anti-reflective performances on glass or polymer transparent covers. The laminated (e.g., sticker) configuration described herein may allow the antireflective material to be fabricated separately from the transparent cover, which may allow the formation of even complex antireflective designs (e.g., nanostructures) that may include multiple layers deposed on a substrate, even where multiple layers requires higher temperatures for formation. By fabricating the anti-reflective sticker with an antireflective coating separately from the transparent cover, this may avoid damaging the transparent cover, and in particular, may avoid heat damage to polymeric transparent covers. In general, these methods and device may have very high performance antireflective coatings.

The anti-reflective materials described herein may have a very high antireflection performances of over wide spectral range and wide angle of incidence range, particularly as compared to other antireflective coating methods such as layer deposition.

The anti-reflective material may be on the outside of the transparent cover (e.g., facing the patient. In some variations, the antireflective material may be on the inside of the transparent cover (e.g., facing the intraoral scanner; in some variations an anti-reflective material may be on both the inside and outside of the transparent cover. It may be particularly advantageous to include the antireflective (e.g., "moth eye") material on the outside of the transparent cover, as it may also be hydrophobic, and may help keep saliva from spreading on the transparent cover.

In some variations the device may include a transparent cover (e.g., on the sleeve/removable barrier for the intraoral scanner) onto which the antireflective material (e.g., a moth eye nanostructured material) is attached. The nanostructured antireflective material (which may be configured to pass both visible light and/or near infrared light) may be laminated on the transparent cover. For example, the moth eye (nanostructured antireflective material) may be configured as a sticker; the sticker may be laminated to a molded sleeve in both internal and external surfaces. A sticker including the nanostructured surface forming the antireflective (moth eye) material may therefore be attached to both the internal and external surfaces during manufacturing. The transparent cover may then be assembled to the sleeve/removable barrier for the intraoral scanner, as described herein.

Alternatively, in some variations, the method of forming the transparent cover of the sleeve may include forming the nanostructured antireflective material on the optical transparent cover. For example, in some variations the transparent cover may be formed by an injection molding process in which the mold for injection molding of the transparent cover includes the nanostructure (e.g., a pattern of hexagonal nanoscale projections). For example, the moth eye antireflective nanostructure may be implemented during the injection molding process of the sleeve optical transparent cover, and the transparent cover may then be assembled to sleeve body. In some variations the moth eye antireflective nanostructure may be implemented during the injection molding process of the whole sleeve body.

In addition to the intraoral scanners described herein, these methods and materials (e.g., stickers of nanostructured antireflective materials) may be used as part of any optical system having a similar geometrical arrangement as described herein.

In some examples a nano-imprint may be used to form an AR layer. For example, a liquid lacquer may be added on the on the external surface of the sleeve (e.g., the transparent cover). The nano structure may be applied on the lacquer by nano imprint photolithography.

A nanostructure may be, e.g., a pattern of hexagonal and/or hexagonally arranged projections having a height of between 100 nm and 900 nm (e.g., between 200 nm and 800 nm, between 250 nm and 700 nm), a pitch of between about 20 nm and 1000 nm (e.g., between 30 nm and 900 nm, etc.) and a gap of between about 100 nm and 600 nm (e.g., between about 200 nm and 500 nm, etc.). In some variations multiple layers of the nanoscale pattern (of projections) may be placed atop each other (e.g., 2 layers, 3 layers, etc.); each layer may be different in material (e.g., SiO₂, Al₂O₃, MgF₂, TiO₂, InSb, ZnO, ZrO₂, HgCdTe, Ge, etc.). The nanostructures may be formed one a substrate comprising the same or a different material (e.g., Si, etc.).

In some examples an AR layer or coating may be applied by vacuum deposition. For example, an injected ACS may be inserted into a vacuum chamber and the external surface of the sleeve and/or the transparent covering. In some examples a dielectric layer may be formed by evaporation in this manner. In some examples, the apparatuses may be coated by dip coating. For example a sleeve and/or transparent cover of a sleeve may be coated by being immersed into a coating bath. Either or both internal and external sides may be coated in this manner.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes, the scope of the invention being defined by the wording of the claims.

## Claims

1. A sleeve (1406,1600,1606,1706,1800) for an intraoral scanner, the sleeve comprising:
a hollow body (1602) having a distal end and a proximal end, wherein the distal end is tapered and closed and the proximal end is open to receive an intraoral scanner;
a window opening (1408) on a lateral side of the distal end, wherein the window opening comprises a seating area (1535, 1735) comprising a lip (1763) around the perimeter of the window opening wherein the lip is formed into the hollow body so that the lip is recessed into an outer surface of the hollow body; and
a transparent cover (1310,1410,1510,1610,1710) over the window opening wherein the transparent cover is configured to fit the seating area so that it may be seated onto the seating area from outside of the sleeve.

2. The sleeve of claim 1, further comprising a ramp within the hollow body, wherein the ramp (633,733) is positioned within the hollow body proximal to the window opening, and configured to guide a distal end of the intraoral scanner away from the window opening as the intraoral scanner is inserted into the sleeve.

3. The sleeve of claim 2, wherein the ramp (633) is adhesively secured to a wall of the hollow body.

4. The sleeve of claim 1, further comprising a gasket (1618) at least partially circumferentially around the window opening between the transparent cover (1610) and the lip (1763),
wherein the transparent cover is secured against the gasket.

5. The sleeve of claim 4, wherein the gasket (1618) comprises a foam material.

6. The sleeve of claim 1, wherein the tapered distal end has a height of between 10 and 16 mm.

7. The sleeve of claim 1, further comprising an anti-reflective coating on both an inner and outer surface of the transparent cover (1310,1410,1510,1610,1710).

8. The sleeve of claim 1, further comprising one or more projections (312) from a periphery of the window opening into the window opening configured to impinge into a field of view of the intraoral scanner when the intraoral scanner is engaged with the sleeve.

9. The sleeve of claim 1, further comprising a sleeve extension (819,855) extending from the proximal end of the hollow body.

10. The sleeve of claim 9, wherein the sleeve extension is configured to fit over the hollow body.

11. The sleeve of claim 9, wherein the sleeve extension is configured to insert into the hollow body.

12. The sleeve of claim 1 wherein the sleeve is formed of a polyethylene material.

13. The sleeve of claim 1, further comprising an adhesive seal (1683) around the window opening sealing the transparent cover to the window opening.

14. The sleeve of claim 1, wherein the transparent cover is flush with an outer surface of the hollow body.

15. The sleeve of claim 1, wherein the transparent cover is ultrasonically welded to the hollow body.

## Patentansprüche

1. Hülse (1406, 1600, 1606, 1706, 1800) für einen Intraoralscanner, wobei die Hülse Folgendes umfasst:
einen Hohlkörper (1602) mit einem distalen Ende und einem proximalen Ende, wobei das distale Ende verjüngt und geschlossen ist und das proximale Ende offen ist, um einen Intraoralscanner aufzunehmen;
eine Fensteröffnung (1408) an einer seitlichen Seite des distalen Endes, wobei die Fensteröffnung (1763) einen Sitzbereich (1535, 1735) umfasst, der eine Lippe (1763) um den Umfang der Fensteröffnung umfasst, wobei die Lippe in den Hohlkörper derart ausgebildet ist, dass die Lippe in eine Außenoberfläche des Hohlkörpers eingelassen ist; und
eine transparente Abdeckung (1310, 1410, 1510, 1610, 1710) über der Fensteröffnung, wobei die transparente Abdeckung konfiguriert ist, um auf den Sitzbereich zu passen, sodass sie von der Außenseite der Hülse auf den Sitzbereich aufgesetzt werden kann.

2. Hülse nach Anspruch 1, ferner umfassend eine Rampe innerhalb des Hohlkörpers, wobei die Rampe (633, 733) innerhalb des Hohlkörpers proximal zu der Fensteröffnung positioniert ist und konfiguriert ist, um ein distales Ende des Intraoralscanners von der Fensteröffnung wegzuführen, wenn der Intraoralscanner in die Hülse eingeführt wird.

3. Hülse nach Anspruch 2, wobei die Rampe (633) an einer Wand des Hohlkörpers klebend gesichert ist.

4. Hülse nach Anspruch 1, ferner umfassend eine Dichtung (1618) wenigstens teilweise um die Fensteröffnung zwischen der transparenten Abdeckung (1610) und der Lippe (1763) umlaufend,
wobei die transparente Abdeckung gegen die Dichtung gesichert ist.

5. Hülse nach Anspruch 4, wobei die Dichtung (1618) ein Schaumstoffmaterial umfasst.

6. Hülse nach Anspruch 1, wobei das verjüngte distale Ende eine Höhe zwischen 10 und 16 mm aufweist.

7. Hülse nach Anspruch 1, ferner umfassend eine Antireflexionsbeschichtung sowohl auf einer Innen- als auch auf einer Außenoberfläche der transparenten Abdeckung (1310, 1410, 1510, 1610, 1710).

8. Hülse nach Anspruch 1, ferner umfassend einen oder mehrere Vorsprünge (3l2) von einem Umfang der Fensteröffnung in die Fensteröffnung, die konfiguriert sind, um auf ein Sichtfeld des Intraoralscanners aufzutreffen, wenn der Intraoralscanner in die Hülse eingreift.

9. Hülse nach Anspruch 1, ferner umfassend eine Hülsenverlängerung (819, 855), die sich von dem proximalen Ende des Hohlkörpers erstreckt.

10. Hülse nach Anspruch 9, wobei die Hülsenverlängerung konfiguriert ist, um über den Hohlkörper zu passen.

11. Hülse nach Anspruch 9, wobei die Hülsenverlängerung konfiguriert ist, um in den Hohlkörper eingesetzt zu werden.

12. Hülse nach Anspruch 1**,** wobei die Hülse aus einem Polyethylenmaterial ausgebildet ist.

13. Hülse nach Anspruch 1, ferner umfassend eine Klebedichtung (1683) um die Fensteröffnung, die die transparente Abdeckung an der Fensteröffnung abdichtet.

14. Hülse nach Anspruch 1, wobei die transparente Abdeckung bündig mit einer Außenoberfläche des Hohlkörpers abschließt.

15. Hülse nach Anspruch 1, wobei die transparente Abdeckung mittels Ultraschall an dem Hohlkörper verschweißt ist.

## Revendications

1. Gaine (1406, 1600, 1606, 1706, 1800) pour un numériseur intrabuccal, la gaine comprenant :
un corps creux (1602) comportant une extrémité distale et une extrémité proximale, l'extrémité distale étant effilée et fermée et l'extrémité proximale étant ouverte pour recevoir un numériseur intrabuccal ;
une ouverture formant fenêtre (1408) sur un côté latéral de l'extrémité distale, l'ouverture formant fenêtre comprenant une région d'appui (1535, 1735) comprenant une lèvre (1763) autour du périmètre de l'ouverture formant fenêtre, la lèvre étant formée dans le corps creux de telle sorte que la lèvre soit en renfoncement dans une surface extérieure du corps creux ; et
un couvercle transparent (1310, 1410, 1510, 1610, 1710) sur l'ouverture formant fenêtre, le couvercle transparent étant conçu pour correspondre à la région d'appui de telle sorte qu'il puisse être placé en appui sur la région d'appui depuis l'extérieur de la gaine.

2. Gaine selon la revendication 1, comprenant, en outre, un plan incliné à l'intérieur du corps creux, dans laquelle le plan incliné (633, 733) est placé à l'intérieur du corps creux à proximité de l'ouverture formant fenêtre, et conçu pour guider une extrémité distale du numériseur intrabuccal à l'écart de l'ouverture formant fenêtre à mesure que le numériseur intrabuccal est inséré dans la gaine.

3. Gaine selon la revendication 2, dans laquelle le plan incliné (633) est fixé par adhérence à une paroi du corps creux.

4. Gaine selon la revendication 1, comprenant, en outre, un joint d'étanchéité (1618) s'étendant au moins partiellement sur la circonférence autour de l'ouverture formant fenêtre entre le couvercle transparent (1610) et la lèvre (1763),
dans laquelle le couvercle transparent est fixé contre le joint d'étanchéité.

5. Gaine selon la revendication 4, dans laquelle le joint d'étanchéité (1618) comprend un matériau sous forme de mousse.

6. Gaine selon la revendication 1, dans laquelle l'extrémité distale effilée présente une hauteur comprise entre 10 et 16 mm.

7. Gaine selon la revendication 1, comprenant, en outre, un revêtement antireflet à la fois sur la surface intérieure et la surface extérieure du couvercle transparent (1310, 1410, 1510, 1610, 1710).

8. Gaine selon la revendication 1, comprenant, en outre, une ou plusieurs saillies (3l2) s'étendant depuis une périphérie de l'ouverture formant fenêtre dans l'ouverture formant fenêtre, conçues pour dépasser dans un champ de vision du numériseur intrabuccal lorsque le numériseur intrabuccal est introduit dans la gaine.

9. Gaine selon la revendication 1, comprenant, en outre, un prolongateur de gaine (819, 855) s'étendant à partir de l'extrémité proximale du corps creux.

10. Gaine selon la revendication 9, dans laquelle le prolongateur de gaine est conçu pour s'adapter par-dessus le corps creux.

11. Gaine selon la revendication 9, dans laquelle le prolongateur de gaine est conçu pour s'adapter dans le corps creux.

12. Gaine selon la revendication 1, la gaine étant composée d'un matériau de polyéthylène.

13. Gaine selon la revendication 1, comprenant, en outre, un élément d'étanchéité adhésif (1683) autour de l'ouverture formant fenêtre reliant de manière étanche le couvercle transparent à l'ouverture formant fenêtre.

14. Gaine selon la revendication 1, dans laquelle le couvercle transparent est de niveau avec une surface extérieure du corps creux.

15. Gaine selon la revendication 1, dans laquelle le couvercle transparent est soudé par ultrasons au corps creux.
